# EUROPÄISCHE PATENTANMELDUNG

(11) **EP 0 555 180 A1**
(43) Veröffentlichungstag der Anmeldung: **11.08.1993**
(21) Anmeldenummer: 93810055.9
(22) Anmeldetag: 27.01.1993
(51) Int. Cl.: C07D 251/22

(54) **Verfahren zur Herstellung von 2-Hydroxy-4,6-diaryl-1,3,5-triazinen**

(30) Priorität: 04.02.1992 CH 323/92
(71) Anmelder: CIBA-GEIGY AG, CH-4002 Basel (CH)
(72) Erfinder: Reinehr, Dieter, Dr., W-7842 Kandern (DE); Bacher, Jean-Pierre, F-68220 Buschwiller (FR)

(57) **Zusammenfassung**

Beschrieben wird ein Verfahren zur Herstellung von 2-Hydroxy-4,6-diaryl-1,3,5-triazinen, das dadurch gekennzeichnet ist, dass man die Verbindung der Formel 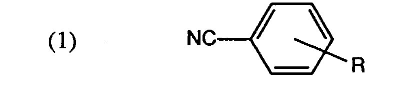
worin
R Wasserstoff, C₁-C₄Alkyl oder Halogen bedeutet,
mit Harnstoff unter Verwendung einer Base, ausgewählt aus der Klasse der Alkalimetall- oder Erdalkalimetallhydride oder Alkali- oder Erdalkaliamide oder der C₁-C₄Alkalialkoholate, in Anwesenheit eines polaren Lösungsmittel zu den Endprodukten in einem einstufigen Verfahren umsetzt und wobei das Molverhältnis Harnstoff zu Base 1 :2 bis 1 :3 und
das Molverhältnis Verbindung der Formel (1)zu Harnstoff 2 :1 bis 5 :1 ist.

Mit dem vorliegenden Verfahren lassen sich 2-Hydroxy-4,6-diaryl-1,3,5-triazine in sehr guten Ausbeuten darstellen. Die Verbindungen finden Verwendung als Zwischenprodukte für die Herstellung von UV-Absorbern.

## Beschreibung

Die vorliegende Erfindung betrifft ein neues, einfaches Verfahren zur Herstellung von 2-Hydroxy-4,6- diaryl-1,3,5-triazinen ausgehend von Harnstoff und aromatischen Nitrilen.

Die Herstellung von 2-Hydroxy-4,6-diaryl-1,3,5-triazinen aus Harnstoff und aromatischen Nitrilen ist bekannt, z.B. aus J. Heterocyclic Chem. 11, 917 (1976). In dieser Referenz wird für die Umsetzungsreaktion als Base Natriumhydrid eingesetzt. Die dabei eingesetzten molaren Mengen sind stets kleiner als die entsprechenden Mengen an Harnstoff.

Überraschenderweise wurde nun gefunden, dass beim Einsatz von zum Harnstoff äquivalenten oder größeren Mengen der Base deutlich höhere Ausbeuten erzielt werden.

Gegenstand der vorliegenden Erfindung ist demnach ein Verfahren zur Herstellung von 2-Hydroxy-4,6- diaryl-1,3,5-triazinen, das dadurch gekennzeichnet ist, dass man die Verbindung der Formel 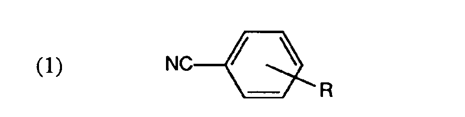
worin
R Wasserstoff, C₁-C₄Alkyl oder Halogen bedeutet,
mit Harnstoff unter Verwendung einer Base, ausgewählt aus der Klasse der Alkalimetall-, Erdalkalimetallhydride oderAlkali-, Erdalkaliamide oder der Alkali-C₁-C₄alkoholate, in Anwesenheit eines polaren Lösungsmittels zu den Endprodukten in einem einstufigen Verfahren umsetzt und wobei
das Molverhältnis Harnstoff zu Base 1:2 bis 1:3 und
das Molverhältnis Verbindung der Formel (1) zu Harnstoff 2:1 bis 5:1 ist.
C₁-C₄Alkyl bei der Definition von R stellt solche Gruppen oder Gruppenbestandteile dar, die 1 bis 4, insbesondere 1 bis 3 Kohlenstoffatome aufweisen. Beispiele für derartige Gruppen sind Methyl, Ethyl, n-Propyl, Isopropyl, n-Butyl, sek.Butyl, tert.Butyl, Amyl, Isoamyl oder tert.Amyl.
Halogen bedeutet insbesonders Fluor, Brom oder Chlor.

Als Beispiele fürAlkalimetalle seien Lithium, Natrium oder Kalium genannt. Bevorzugt ist Natrium. Beispiele für Erdalk alimetalle sind Calcium und Magnesium.

Vorzugsweise kommen Verbindungen der Formel (1) in Betracht, bei denen R Wasserstoff, Methyl oder Chlor bedeutet.

Ganz besonders bevorzugt sind solche Verbindungen der Formel (1), bei denen Methyl und Chlor nicht orthoständig zur Cyano-Gruppe stehen.

Als Beispiele für die erfindungsgemäss eingesetzten Verbindungen der Formel (1) seien Benzonitril, m-Tolunitril, p-Tolunitril, p-Chlorbenzonitril oder m-Chlorbenzonitril genannt.

Als Beispiel für die erfindungsgemäss eingesetzten Basen seien Lithiumhydrid, Natriumhydrid, Kaliumhydrid, Lithiumamid, Natriumamid, Natriummethanolat oder Natriumethanolat genannt.

Die Umsetzung der Verbindung der Formel (1) mit Harnstoff erfolgt in Anwesenheiteines polaren Lösungsmittels, wie beispielsweise Dimethylsulfoxid, Dimethylformamid oder Hexamethylphosphorsäuretriamid. Vorzugsweise wird als polares Lösungsmittel Dimethylsulfoxid verwendet.

Die Reaktionsdauer liegt zwischen 2 und 24 Stunden, vorzugsweise 2 und 8 Stunden, die Reaktionstemperatur zwischen 20 und 70°C, vorzugweise zwischen 30 und 60°C.

In einer besonders bevorzugten Ausführungsform des erfindungsgemässen Verfahrens wird die Verbindung der Formel 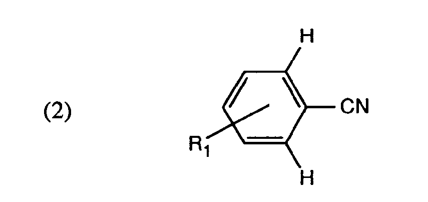
worin
R₁ Methyl oder Chlor bedeutet, mit Harnstoff unter Verwendung von Natriumamid in Anwesenheit von Dimethylsulfoxid zu den Endprodukten in einem einstufigen Verfahren umgesetzt, wobei das Molverhältnis Harnstoff zu Base 1:2 bis 1:3 und
das Molverhältnis Verbindung der Formel (2) zu Harnstoff 2:1 bis 5:1 ist.

Mit dem vorliegenden Verfahren lassen sich 2-Hydroxy-4,6-diaryl-1,3,5-triazine in guten Ausbeuten und hoher Reinheit herstellen. Gegenüber den bekannten Verfahren lässt sich die Umsetzungsreaktion bei niedrigeren Temperaturen durchführen. Die nach dem erfindungsgemässen Verfahren hergestellten Verbindungen sind Zwischenprodukte für die Herstellung von UV-Absorbern.

Die folgenden Beispiele veranschaulichen die Erfindung.

### Beispiel 1: Herstellung von 2-Hydroxy-4,6-diphenyl-1 ,3,5-triazin mit Natriumamid

6 g (0.1 Mol) Harnstoff und 41,2 g (0,4 Mol) Benzonitril werden bei 20°C in 100 ml Dimethylsulfoxid (DMSO) vorgelegt. Darauf werden unter Rühren 6 g (0,2 Mol) Natriumhydrid (80%ig in Mineralöl) zugegeben, wobei unter Schaumbildung die Temperatur der Mischung innerhalb von ca. 5 Minuten auf 44°C ansteigt. Dann wird noch 18 Stunden bei Raumtemperatur nachgerührt und auf 200 ml Wasser gegossen, das mit 10 ml Eisessig angesäuert wird. Das ausgefallene Produkt wird abgesaugt und getrocknet (25,8 g eines hellbeigen Produktes). Nach der Umkristallisation aus 110 ml Dimethylformamid erhält man 20 g der Verbindung der Formel 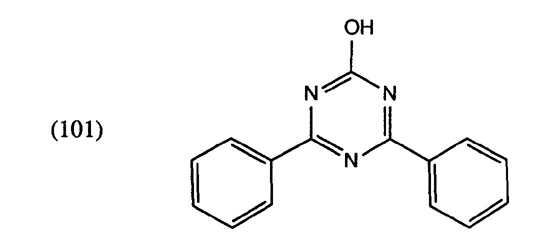
die als fast farbloses Pulver anfällt.
Ausbeute: 80% d. Theorie
Fp.: 297-298°C

### Elementaranalyse:

Gefunden 72,33% C; 4,63% H; 16,96% N
Berechnet als C₁₅H₁₁N₃O: 72,28% C; 4,55% H; 16,86% N;

### Beispiel 2: Herstellung von 2-Hydroxy-4,6-diphenyl-1 ,3,5-triazin mit Natriumamid

12 g (0,2 Mol) Harnstoff wird in 200 ml Dimethylsulfoxid gelöst und mit 21,6 g (0,5 Mol) Natriumamid (90%ig) versetzt. Die leicht exotherme Reaktion lässt die Temperatur auf 30°C ansteigen. Es wird dann weiter auf 50°C erwärmt und während 18 Stunden bei dieser Temperatur gerührt. Man kühlt auf Raumtemperatur ab, versetzt mit 82,4 g (0,8 Mol) Benzonitril und rührt während 4 Stunden bei 50°C. Es wird dann mit 500 ml Methanol verdünnt, mit Eisessig angesäuert und abfiltriert. Es werden 41 g der Verbindung der Formel (101) erhalten.

Ausbeute: 82,2% d. Theorie

### Beispiel 3: Herstellung von 2-Hydroxy-4,6-di(4-methylphenyl)-1 ,3,5-triazin

Man verfährt wie in Beispiel 2 beschrieben mit dem Unterschied, dass man anstelle von 82,4 g (0,8 Mol) Benzonitril 93,6 g (0,8 Mol) p-Tolunitril verwendet. Nach der gewohnten Aufarbeitung erhält man 38 g eines beigen Produktes, das nach der Umkristallisation aus 370 ml Dimethylformamid 29 g einer hellbeigen Verbindung der Formel 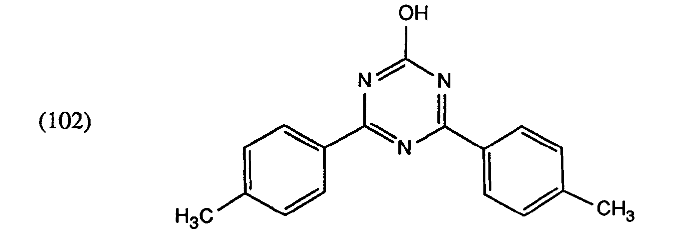
ergibt.
Ausbeute: 68,5 % d. Theorie
Fp.: >300_{°}C

### Elementaranalyse:

Gefunden 73,15% C; 5,49% H; 15,11% N
Berechnet als C₁₇H₁₅N₃O: 73,63% C; 5,45% H; 15,15% N;

### Beispiel 4: Herstellung von 2-Hydroxy-4,6-di(3-methylphenyl)-1 ,3,5-triazin

Man verfährt wie in Beispiel 2 beschrieben mit dem Unterschied, dass man anstelle von 82,4 g (0,8 Mol) Benzonitril 93,6 g (0,8 Mol) m-Tolunitril verwendet. Nach der gewohnten Aufarbeitung erhält man 38,5 g eines hellen Produktes, das nach der Umkristallisation aus Methylcellusolve 34,2 g einer hellen Verbindung der Formel 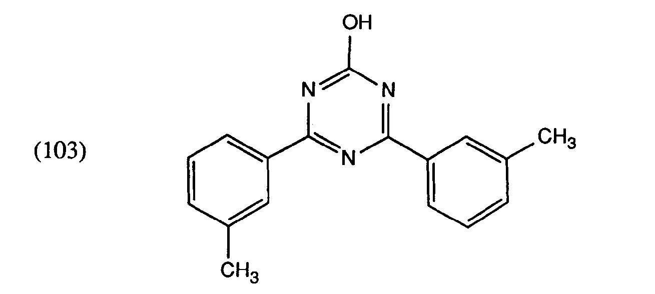
ergibt.
Ausbeute: 61,7 % d. Theorie
Fp.: 234-234,5°C

### Elementaranalyse:

Gefunden 72,84% C; 5,46% H; 15,24% N
Berechnet als C₁₇H₁₅N₃O: 73,63% C; 5,45% H; 15,15% N; 5,77% O

### Beispiel 5: Herstellung von 2-Hydroxy-4,6-di(4-chlorphenyl)-1 ,3,5-triazin

Man verfährt wie in Beispiel 2 beschrieben mit dem Unterschied, dass man anstelle von 82,4 g (0,8 Mol) Benzonitril 100 g (0,72 Mol) p-Chlorbenzonitril verwendet. Nach der gewohnten Aufarbeitung erhält man 62 g eines hellbeigen Produktes, das nach der Umkristallisation aus Dimethylformamid 50,5 g einer blass-beigen Verbindung der Formel 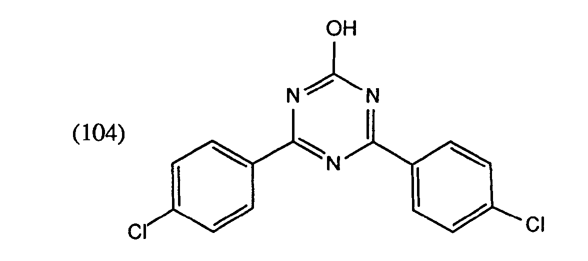
ergibt.
Ausbeute: 79,5 % d. Theorie
Fp.: >300_{°}C

### Elementaranalyse:

Gefunden:56,44% C; 2,84% H; 13,28% N; 22,02% CI
Berechnet als C₁₅H₁₉Cl₂N₃O: 56,63% C; 2,85% H; 13,21% N; 22,29 % Cl,

### Beispiel 6: Herstellung von 2-Hydroxy-4,6-di(3-chlorphenyl)-1 ,3,5-triazin

Man verfährt wie in Beispiel 2 beschrieben mit dem Unterschied, dass man anstelle von 82,4 g (0,8 Mol) Benzonitril 100 g (0,72 Mol) m-Chlorbenzonitril verwendet. Nach der gewohnten Aufarbeitung erhält man 52 g eines hellen Produktes, das nach der Umkristallisation aus Methylcellusolve und Dimethylformamid 27,6 g einer hellbeigen Verbindung der Formel 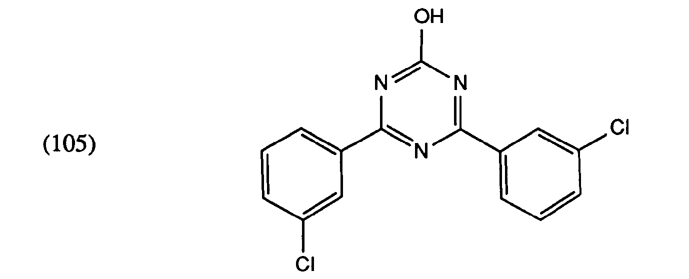
ergibt.
Ausbeute: 43,4 % d. Theorie
Fp.: 286-287°C

### Elementaranalyse:

Gefunden 55,91% C; 2,98% H; 13,14% N; 21,63% CI
Berechnet als C₁₅H₁₉Cl₂N₃O: 56,63% C; 2,85% H; 13,21% N; 22,29% CI

## Patentansprüche

1. Verfahren zur Herstellung von 2-Hydroxy-4,6-diaryl-1,3,5-triazinen, dadurch gekennzeichnet, dass man die Verbindung der Formel 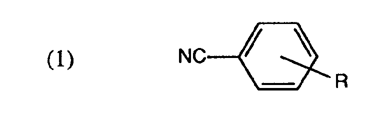
worin
R Wasserstoff, C₁-C₄Alkyl oder Halogen bedeutet,
mit Harnstoff unter Verwendung einer Base, ausgewählt aus der Klasse der Alkalimetall- oder Erdalkalimetallhydride oder Alkali- oder Erdalkaliamide oder der C₁-C₄Alkalialkoholate, in Anwesenheit eines polaren Lösungsmittels zu den Endprodukten in einem einstufigen Verfahren umsetzt und wobei das Molverhältnis Harnstoff zu Base 1:2 bis 1:3 und
das Molverhältnis Verbindung der Formel (1)zu Harnstoff 2:1 bis 5:1 ist.

2. Verfahren gemäss Anspruch 1, dadurch gekennzeichnet, dass in Formel (1) R Wasserstoff, Methyl oder Chlor bedeutet.

3. Verfahren gemäss Anspruch 2, dadurch gekennzeichnet, dass Methyl und Chlor nicht orthoständig zur Cyano-Gruppe stehen.

4. Verfahren gemäss einem der Ansprüche 1 bis 3, dadurch gekennzeichnet, dass die Reaktion bei einer Temperatur zwischen 20 und 70°C durchgeführt wird.

5. Verfahren gemäss einem der Ansprüche 1 bis 4, dadurch gekennzeichnet, dass die Reaktion innerhalb von 2 bis 24 Stunden durchgeführt wird.

6. Verfahren gemäss einem der Ansprüche 1 bis 5, dadurch gekennzeichnet, dass als polares Lösungsmittel Dimethylsulfoxid, Dimethylformamid oder Hexamethylphosphorsäuretriamid verwendet wird.

7. Verfahren gemäss einem der Ansprüche 1 bis 6, dadurch gekennzeichnet, dass man die Verbindung der Formel 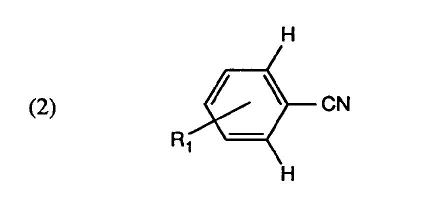
worin
R₁ Methyl oder Chlor bedeutet, mit Harnstoff unter Verwendung von Natriumamid in Anwesenheit von Dimethylsulfoxid zu den Endprodukten in einem einstufigen Verfahren umsetzt, wobei
das Molverhältnis Harnstoff zu Base 1:2 bis 1:3 und
das Molverhältnis Verbindung der Formel (2) zu Harnstoff 2:1 bis 5:1 ist.

8. Verwendung der gemäss Anspruch 1 hergestellten 2-Hydroxy-4,6-diaryl-1,3,5-triazine als Zwischenprodukte für die Herstellung von UV-Absorbern.
